# EUROPEAN PATENT SPECIFICATION

(11) **EP 0 550 585 B1**
(45) Date of publication and mention of the grant of the patent: **10.08.1994**
(21) Application number: 91917298.1
(22) Date of filing: 25.09.1991
(51) Int. Cl.: A61B 5/14

(54) **Lancet device**
Lanzette-Vorrichtung
Lancette

(30) Priority: 25.09.1990 DK 2316/90
(43) Date of publication of application: 14.07.1993
(73) Proprietor: BRITISH TECHNOLOGY GROUP LIMITED, London SE1 6BU (GB)
(72) Inventor: POULSEN, Jens, Ulrik, DK-2200 Copenhagen N. (DK); LARSEN, Andre, DK-2791 Dragoer (DK); MOLLER-JENSEN, Jens, DK-1051 Copenhagen K. (DK)
(74) Representative: Chandler, Derek Richard
(86) International application number: DK9100289
(87) International publication number: WO9204866

(56) References cited:
- EP-A- 0 115 388
- EP-A- 0 255 338
- EP-A- 0 414 563
- EP-A- 0 427 406
- US-A- 4 462 405
- US-A- 4 469 110
- US-A- 4 553 541
- US-A- 4 580 565
- US-A- 4 658 821

## Description

The invention relates to a lancet device comprising a tool having a lancet drive hammer which may be cocked and released, and holding means for holding a disposable lancet and a finger plate with an opening through which the lancet may be driven when the lancet drive is released for piercing the skin on the location where the sample should be taken.

By medical examinations it is often necessary to take out small amounts of blood of the order of a single droplet. These samples are provided by piercing the skin of the patient so that a single drop of blood trickles out.

Preferred for this purpose is a tool by which a stiletto or a needle by an impact is quickly forced through the skin and by which the needle before and after the operation is hidden to the patient, as this may be an advantage seen from a psychological point of view. The retraction of the needle also prevents the user from scratching himself or other persons with the used needle, which may be infected.

Some patients e.g. patients suffering from diabetes or from haemophilia have to take out blood samples from themselves frequently to estimate their need for medication. For such patients it is of importance that the samples can be taken in a simple way without pain using a tool with a neutral appearance, and which is simple to handle.

EP-A-0,255,338 discloses a lancet device for performing a pricking operation. The device is a disposable one wherein a cocked spring may be released to force a stiletto into the skin and thereupon retract it into the housing of the device. The device is a simple disposable one wherein the stiletto may not be changed, and the spring may not be recocked. Further, the device allows no adjusting of the pricking depth.

US-A-4,462,405 discloses a lancet device wherein only the stiletto is disposable, whereas the rest of the device constitutes a reusable tool having a releasable lancet drive hammer and holding means for the disposable lancet."

A tool is known having the appearance of a cigarette lighter in which a needle unit may be placed and by a loading motion set under influence of a compressed spring which may be released by pressing a button, whereby the needle is driven through an opening in a finger plate on which the patient has positioned his finger. Thereby the finger is perforated by the needle to a depth regulated by the choice of interchangeable finger plates. After use the needle can be ejected from the tool by pressing another button.

With the latest attention to blood transferred diseases, it is desirable to make it possible to throw away after use parts which may have been in contact with blood. The disposal of the relevant parts should be possible without touching such parts. By the lighter model these demands are met as to the rejection of the needle, whereas the finger plates are not intended to be disposable.

Consequently, the object of this invention is to provide a lancet device which makes sure that needle as well as finger plate may be disposed of after use without having to touch either of the parts.

This is obtained by the lancet and the finger plate being integrated in a lancet unit to be received in a socket constituting the holding means of the tool, in which unit a stiletto having a pointed front end and a rear end is resiliently suspended behind the finger plate in a suspension rigidly connected with this finger plate and allowing displacement of the stiletto in its axial direction perpendicular to the finger plate so that the pointed end is driven to protrude from the front side of the finger plate, when the stiletto on its rear end receives an axial impact from the released lancet drive hammer, and is retracted to its initial position behind the finger plate when the impact influence has ceased.

By this construction with the finger plate, the stiletto and its suspension mutually ridigly connected to each other it is ensured that needle and finger plate are disposed of at the same time.

According to a further embodiment of the invention, the resilient suspension of the stiletto may be formed by a meandering leaf spring, the elasticity of which varies along the leaf in such a way that it is ensured that the stiletto does not or only to a minor degree diverge from its direction perpendicular to the finger plate when influenced by an axial impact on its rear end. Without this precaution the stiletto may pierce the skin at an oblique angle causing a scratching instead of a pure sting, thus making the piercing more painful or insufficient.

In a preferred embodiment according to the invention, the stiletto is covered by a protecting cover, which is fastened to the stiletto suspension and has a finger grib accessible on the front side of the finger plate for twisting the cover free and removing it before use when the lancet unit is placed in a tool for use.

By moulding the finger plate, suspension, and protecting cover as an integral piece, in which the stiletto is embedded by the mould procedure, the rigid fastening of the protecting cover is secured, and further the stiletto is sterilized by the heat of the embedding material during the mould procedure.

According to a preferred embodiment of the invention the tool may comprise a rod shaped lancet drive hammer guided in a tube coaxially with a housing, this tube having a diameter allowing the hammer rod to lie obliguely in relation to the axis of the tube; a loading mechanism forcing the hammer away from the socket end of the housing against the force of a shot spring to a position centred in the tube with the shot spring tightened and held by a releasable pawl mechanism with an end of the hammer inserted in a bore in an operation button coaxial with the housing at the end opposite the socket; an ejector inside the socket comprising a block covering the orifice of the tube at the end facing the socket and having central opening, through which the hammer may just pass when centred in the tube; means for forcing the hammer into an oblique position in the tube with one end adjacent to the ejector block beside its opening, and the other end adjacent to the inner end of the operation button beside its bore; and adjustable means for restricting the lengthwise movement of the hammer when it is released for impacting a rear end of the stiletto of the lancet unit inserted in the socket.

According to a preferred embodiment of the invention the loading mechanism of the tool may comprise a block rotably mounted on the tube end facing the socket and having an outer contour mating with an inner contour in an independent part of the housing comprising the socket to be turned with this part of the housing, the tube being provided at opposite sides with slots, into which protrude the ends of a pin through the hammer, perpendicular to its axis and secured in the hammer with equal parts protruding at each side of the hammer, and the block being provided with corresponding slots, each of which at the position for the pin, when the hammer is in its neutral position, pass over into a helical ramp extending half the way around the block, the first part of the ramp having guides for influencing the pin in its longitudinal direction to centre the hammer in the tube before it is moved against the force of the shot spring by the ends of the pin sliding upwards the ramps when the block is rotated on the tube.

According to a preferred embodiment of the invention the adjustable means for limiting the movement of the hammer may comprise a sleeve rotably mounted in the housing coaxial with this housing at the end opposite the socket, the sleeve being coupled to the button to be rotated by turning this button and having a stop mechanism to keep it in a selected rotary position, and a bottom plate provided at the inner end of the sleeve, through which bottom plate the hammer passes, this bottom plate having on its side facing the inner of the sleeve an angular stepped face with progressively increasing heighth of the steps, which face cooperates with a stop pin on the hammer inside the sleeve to restrict the movement of the hammer when released, each step corresponding to a selectable rotary position of the sleeve.

The invention is now described with reference to the accompanying drawings, in which

Figure 1 shows an end view of a lancet unit according to the invention.

Figure 2 shows a top plan view of the lancet unit of figure 1.

Figure 3 shows a side view of the lancet unit of figure 1.

Figure 4 shows a cross-section taken along the line IV-IV of figure 2.

Figure 5 shows the lancet unit of figure 1 seen from the bottom.

Figure 6 shows a perspective view of a tool for operating the lancet unit of figures 1-5.

Figure 7 shows the tool of figure 6 seen from the socket end.

Figure 8 shows a sectional view along the longitudinal axis of the tool of figure 6 along the line VIII-VIII in figure 7, the tool being shown unloaded, i.e. with its hammer in its neutral position, and carrying a lancet unit.

Figure 9 shows a sectional view as in figure 8 with the hammer in its neutral position, but with certain details omitted.

Figure 10 shows a sectional view as in figure 9 with the hammer in a loaded position ready to be released.

Figure 11 shows a sectional view as in figure 9 with the hammer released impacting the stiletto of the lancet unit, and

Figure 12 shows a sectional view as in figure 9 with the hammer serving an ejection function.

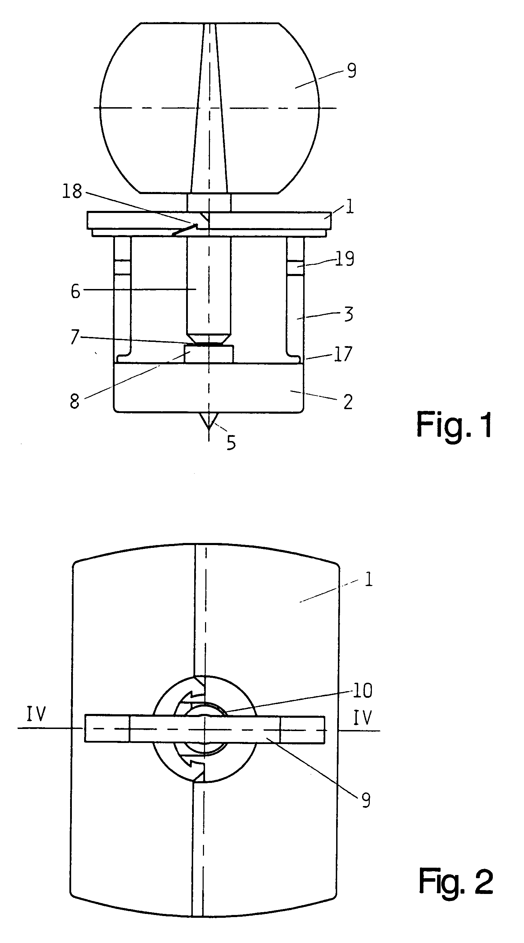

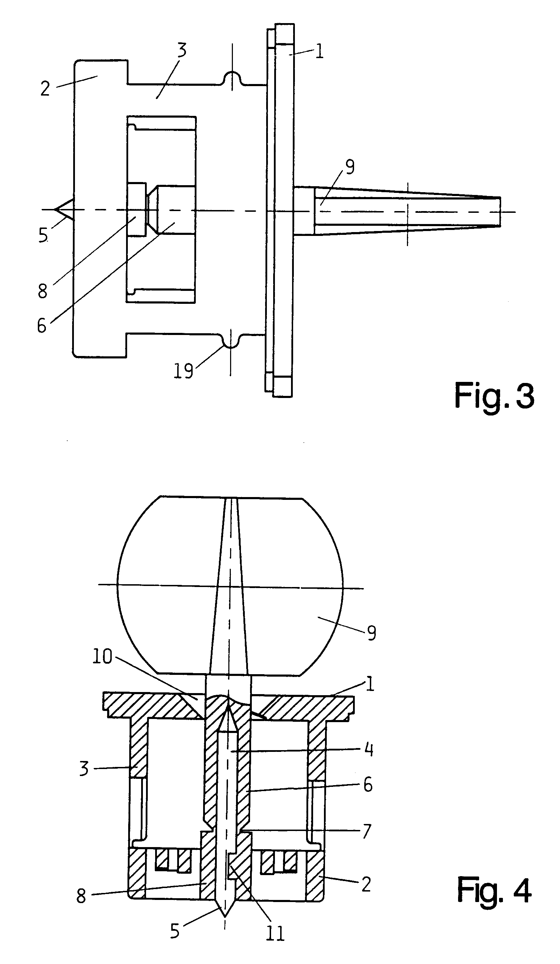

Reference is now made to figures 1-5 illustrating a lancet unit to be used in the lancet device according to the invention. A finger plate 1 is connected to a suspension frame 2 through side walls 3. In the suspension frame 2 the stiletto 4 is suspended, but in figures 1 and 3 only the rear end 5 of this stiletto 4 is seen, the pointed end being covered by a protecting cover 6, which by a weakened part 7 is fastened to the embedment 8, where the stiletto 4 is embedded in its suspension. At the front side of the finger plate 1 the protecting cover 6, extending through an opening 10 in the finger plate 1, is provided with a finger grip 9, by which the cover may be twisted to break the weakened part 7 to remove the cover from the stiletto. This removal of the cover is not performed until the lancet unit is placed with its suspension frame and its side walls in a socket in an operating tool, the insertion in the socket being limited by the rear side of the finger plate abutting the front edge of the socket and lateral play being prevented by guiding projections 19 abutting the inner wall of the socket.

Figure 4 illustrates a section along the line IV-IV in figure 2 showing the stiletto 4 in its embedment 8 in the suspension. As can be seen, the stiletto 4 has a recess 11 in its embedded part, whereby it is ensured that the stiletto is not twisted out of its embedment 8 when the protecting cover 6 is twisted to break the weakened part 7.

As can also be seen from figure 4, the pointed end of the stiletto lies ready in a position behind the finger plate 1 when the protecting cover has been removed. An impact on the rear end 5 of the stiletto will force the point of the stiletto up through the opening 10 in the finger plate 1 to pierce a finger placed on this plate. After the piercing, when the impact influence on the rear end 5 of the stiletto 4 has ceased, the spring effect of the suspension will retract the stiletto to a position with its pointed end behind the front side of the finger plate 1.

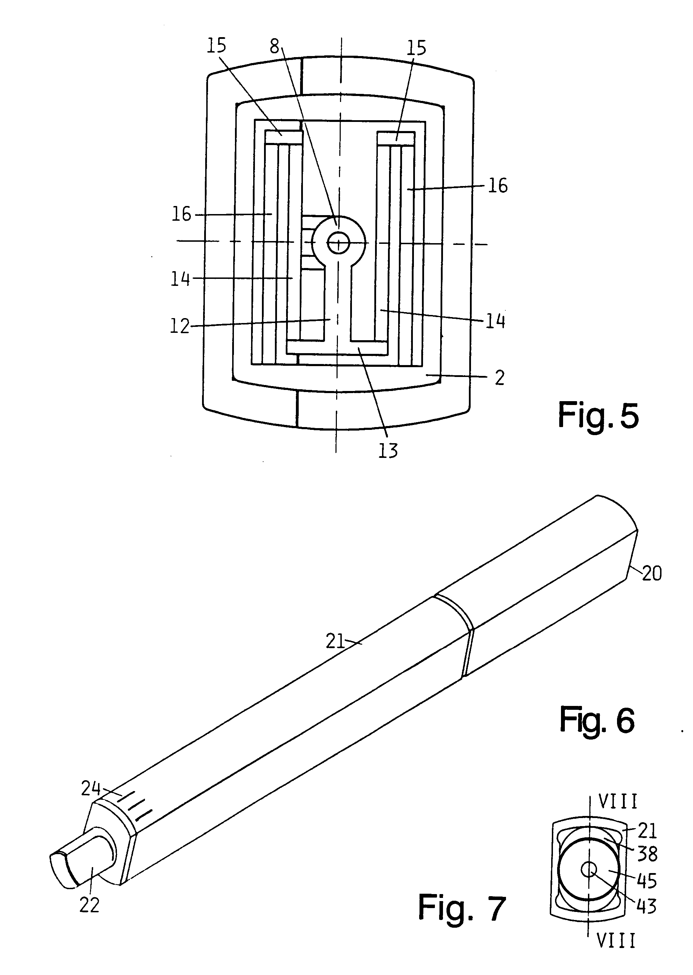

Figure 5, showing the lancet unit seen from the bottom, illustrates the suspension of the stiletto which is embedded in the embedment 8. Through a connecting part 12 this embedment is connected to the middle of a beam 13 having its ends connected through leaf springs 14 each to an end of two connection beams 15, the other ends of which are connected through other leaf springs 16 parallel with the first pair of leaf springs 14 to the part of the suspension frame 2 adjacent to the first beam.

Bending one pair of the leaf springs out of their plane will cause a tilting of the stiletto away from its position perpendicular to the finger plate, whereas bending of the other pair of leaf springs will cause a tilting in the opposite direction. By an appropriate dimensioning of the elasticity of the springs, recognizing that the springs have different lengths, it is ensured that the movement of the stiletto is a pure axial movement.

The shown lancet unit is moulded with the side walls 3 lying in horizontal continuation of the suspension frame 2 with a half part of the finger plate extending upwards of the outer end of each side wall perpendicular to the respective side wall. The connection of the side walls to the suspension frame 2 is formed as hinges 17 permitting the side walls to be folded upwards to make the finger plate halves meet and snap together by a snap lock 18 provided along the meeting edges.

The rear end of the stiletto is preferably blunt, but may have a sharp edge by being cut off by a wire cutter. In another embodiment not shown the rear end of the stiletto may not project from the embedment whereby the plastic material of this embodiment serves as an anvil for an impact mechanism.

The lancet unit may be moulded into its final shape so that the finger plate appears as an integral surface instead of a surface formed by meeting halves.

Further, the protecting cover may be omitted and the lancet units may be packed individually in sterile packages.

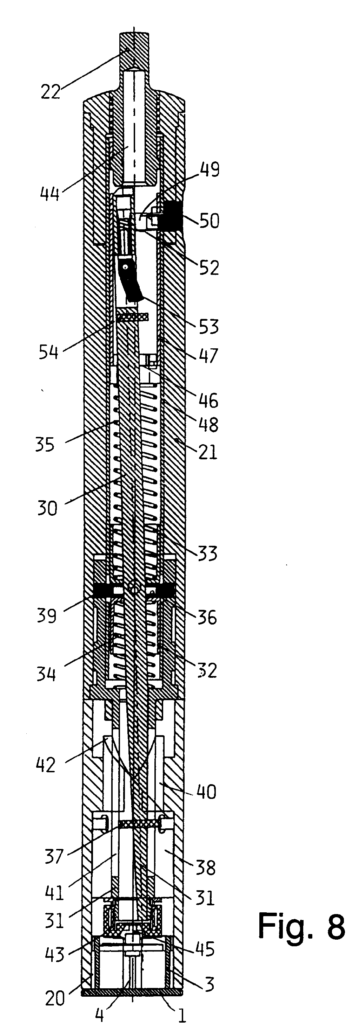

The lancet unit is operated using a pencil-shaped tool as shown in figures 6-12. The tool comprises a housing 21 having a mainly rectangular cross-section and having at one end a socket 20 for receiving the lancet unit and at the other end a button 22, which may be pressed or turned for different operation purposes.

The end involving the socket 20 can be turned about its axis, and a turning of this end 180° in relation to the rest of the housing will tighten a shot spring 35 for shooting a hammer 30 against the rear end of the stiletto of lancet unit inserted in the socket 20. When the shot spring 35 is tightened, the button 22 serves as a trigger for releasing the hammer, but when the shot spring 35 is not tightened the button 22 ejects the lancet unit when pressed.

Turning the button 22 about its axis will adjust how far the hammer will be shot, and the piercing depth of the stiletto may thus be adjusted by turning the button to point at one of a number of marks 24 representing various selectable positions. The end of the button has a rectangular cross-section and may thus function as an arrow pointing at the mark corresponding to the chosen position.

The hammer 30 is shaped as a rod extending through the centre of the housing guided at one end in a tube 31 and at 20 about its middle in central openings in two bushings 32 and 33, transferring the forces of a retraction spring 34 and a shot spring 35, respectively, to the hammer 30 by influencing the respective sides of a ring 36 fastened to the hammer 30 at an oblique angle. The bushings 32 and 33 are guided in a tubular lining 48 in the housing.

The tube 31 has in its wall two opposite slots 41 guiding the respective ends of a pin 37 going through the hammer 30 perpendicular to its axis and secured in the hammer 30 in such a way that its ends protrude equally through each side of the tube 31, when the hammer 30 is centred with its axis identic with the axis of the housing.

Turnable on the tube 31 is mounted a tightening block 38 having slots 40 corresponding to the slots 41 of the tube 31, but passing over into a helical ramp 42 extending half way around the block beginning at the position of the pin 37 when the hammer 30 is in its neutral position defined by the ring 36 lying abreast of stops 39 for the bushings 32, 33.

When in its neutral position the hammer 30 will, due to the influence on the ring 36, adabt an oblique position in a plane defined by the slots 41 in the tube. In the directions perpendicular to this plane the position of the hammer is unvariably defined by the openings in the bushings 32, 33, and the pin 37 sliding in the slots 41 in the tube 31.

In its oblique position the hammer will at its one end be adjacent to an edge of an ejector 43 and at its other end be adjacent to an edge of the inner end of the operation button 22, and will consequently transfer an axial movement of the button 22 to the ejector 43 and force this ejector against the suspension frame 2 of a lancet unit in the socket 20 to eject this lancet unit when the button 22 is pressed.

When the turnable tightening block 38 is turned 180° to tighten the shot spring 35, the hammer will initially be centred in the housing, as guides at the beginning of the ramp 42 position the pin with equal protruding parts on each side of the tube 31. Thereupon the pin 37 is moved along the slots by the ramps 42 and the shot spring 35 is tightened by the hammer 30 being moved in the direction towards the operation button. The hammer, now being centred in the housing, will pass with its end into a bore 44 in the operation button 22 and by a pawl mechanism 53 be kept in its position with the shot spring 35 tightened.

When the operation button is now pressed it will release the pawl mechanism 53, and the shot spring will move the hammer 30 towards its neutral position. Due to its momentum the hammer will pass this position, and now being centred it will shoot out through an opening 45 in the ejector 43 and impart an impact on the rear end of the stiletto of a lancet unit inserted in the socket 20 of the tool.

Shooting past its neutral position the hammer 30 will tighten the retraction spring 34 by the ring 36 abutting the bushing 32, and this spring will finally retract the hammer to its neutral position.

The shot spring 35, exerting at its one end a force on the hammer through the bushing 33, abuts at its other end a plate 46 at one end of a sleeve 47, which fits rotably into the tubular lining 48 in the housing. At the other end of the sleeve 47 the inner end of the operation button fits into a guidance, permitting an axial motion of the button, but which guidance ensures that a turning of the button about its axis will impart a turning of the sleeve 47 in the lining 48.

At a position between the ends of the sleeve its wall is cut away leaving a partly roundgoing opening 49, into which a stop pin 50 protrudes inserted through the lining 48 and secured in the housing to prevent the sleeve 47 from being driven out by the force of the shot spring 35. The edge 52 of the opening 49, against which the stop pin 50 abuts, in a plane perpendicular to the axis of the sleeve, is at intervals along its length provided with rounded depressions mating the abutting part of the stop pin 50. When the shot spring 35 acting on the end of the sleeve 47 forces the edge 52 against the stop pin 50, the depressions will, when the tube is rotated, define fixed rotary positions for the sleeve 47.

When the hammer 30 is released and is shooting in the direction towards a lancet unit inserted in the socket 20 of the tool, the movement of the hammer 30 is restricted by a pin 54 on the hammer inside the sleeve 47 hitting the plate 46 on its side facing the inner of the sleeve 47.

The circular inner side of the plate 46 is provided with a stepped face, the angular length of each step being equal to the angular distance between the depressions along the edge 52 of the opening 49 in the sleeve 47. The steps progressively restrict the length of the movement of the hammer 30 by providing abutments for a pin 54 secured in the hammer 30 perpendicularly to its axis and projecting from one side of the hammer. In this way it is made possible to adjust the piercing depth for the stiletto of a lancet unit inserted in the tool.

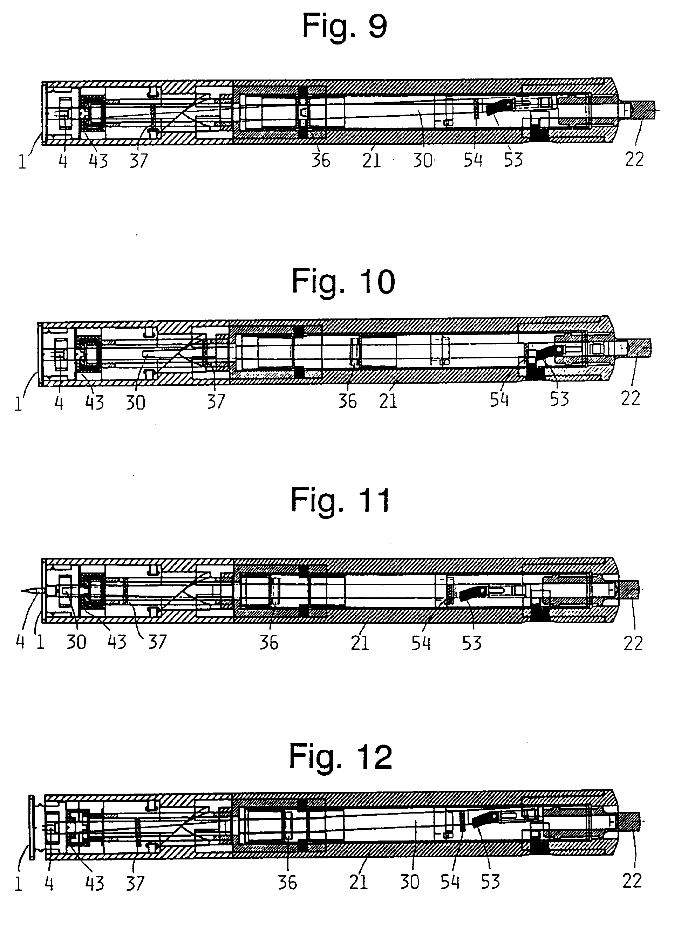

Figures 9-12 illustrate various statuses of a tool with a lancet unit mounted. For the sake of clearnes, some details, e.g. the shot spring and retracting spring, are omitted.

Figure 9 shows the tool in its neutral status. The not shown springs force the bushings 32 and 33 against the stops 39. The ring 36 will be forced to adapt a position perpendicular to the housing axis and consequently the hammer 30 will be forced to an oblique position with its one end facing the edge of the inner end of the operation button 22 and the other facing the ejector 43 away from its central opening 45. In this neutral status the tool may receive a lancet unit in its socket.

In figure 10 the socket end of the housing has been twisted 180° relative to the rest of the housing. Thereby the pin 37 has been passed along the ramps 42 and the hammer 30 is passed to the right. Sliding in the axial slots 41 the pin 37 is maintained in an axial plane in the housing. Guided by not shown guides the pin is moved in this axial plane to center the hammer before passing it to the right. So centered the hammer may be passed into the bore 44 of the operation button 22 and the pawl 53 may engage a stop preventing the shot spring from passing the hammer back to its neutral position.

In figure 11 the button 22 is pressed and the pawl 53 has been forced away from the stop. The shot spring has forced the bushing 33 back to its position abutting the stop 39. Thereby the hammer has been imparted a momentum driving it further to the left to a position in which it hits the rear end of the stiletto of the lancet unit mounted in the socket and forces the stiletto 4 to shoot out through the opening in the finger plate 1. The movement of the hammer is stopped by the pin 54 abutting the bottom plate 46 of the depth setting mechanism. As the momentum of the stiletto and its suspension is negligible the movement of the hammer will define the pricking depth.

After the impact the retraction spring will move the hammer back to its neutral position and the status of figure 9 is reestablished. If the button 22 is now pressed the situation shown in figure 12 occurs where the movement of the button 22 via the oblique hammer is transferred to the ejector 43 which is also moved to the left ejecting the lancet unit from the socket.

## Claims

1. A lancet device comprising a tool having a lancet drive hammer (30) which may be cocked and released, and holding means for holding a disposable lancet and a finger plate (1) with an opening through which the lancet may be driven when the lancet drive is released, **characterized** in, that the lancet and the finger plate (1) are integrated in a lancet unit to be received in a socket (20) constituting the holding means of the tool, in which unit a stiletto (4) having a pointed front end and a rear end (5) is resiliently suspended behind the finger plate (1) in a suspension rigidly connected with this finger plate (1) and allowing displacement of the stiletto (4) in its axial direction perpendicular to the finger plate (1) so that the pointed end is driven to protrude from the front side of the finger plate (1), when the stiletto (4) on its rear end (5) receives an axial Impact from the released lancet drive hammer (30), and is retracted to its initial position behind the finger plate (1) when the impact influence has ceased.

2. A lancet device according to claim 1, **characterized** in that the resilient suspension of the stiletto (4) is formed by a meandering leaf spring (14,15,16), the elasticity of which varies along the leaf in such a way that it is ensured that the stiletto (4) does not or only to a minor extent diverge from its direction perpendicular to the finger plate (1) when influenced by an axial impact on its rear end (5).

3. A lancet device according to claim 1 or 2, **characterized** in that the stiletto (4) is covered by a protecting cover (6) which is fastened to the stiletto suspension and has a finger grib (9) accessible at the front side of the finger plate (1) for twisting the cover (6) free and removing it before use, preferably after the lancet unit is placed in a tool.

4. A lancet device according to claim 1, 2 or 3, **characterized** in that the finger plate (1), suspension and protecting cover (6) are moulded as an integral piece, in which the stiletto (4) is embedded by the mould procedure.

5. A lancet device according to claim 1, **characterized** in, that the tool comprises a rod shaped lancet drive hammer (30) guided in a tube (31) coaxially with a housing (21), this tube having a diameter allowing the hammer rod (30) to lie obliguely in relation to the axis of the tube (31); a loading mechanism forcing the hammer (30) away from the socket end of the housing (21) against the force of a shot spring (35) to a position centred in the tube (31) with the shot spring (35) tightened and held by a releasable pawl mechanism (53) with an end of the hammer (30) inserted in a bore (44) in an operation button (22) coaxial with the housing (21) at the end opposite the socket (20); an ejector (43) inside the socket (20) comprising a block covering the orifice of the tube (31) at the end facing the socket (20) and having central opening (45), through which the hammer (30) may just pass when centred in the tube (31); means for forcing the hammer (30) into an oblique position in the tube (31) with one end adjacent to the ejector block (43) beside its opening (45), and the other end adjacent to the inner end of the operation button (22) beside its bore (44); and adjustable means for restricting the lengthwise movement of the hammer (30) when it is released for impacting a rear end (5) of the stiletto (4) of the lancet unit inserted in the socket (20).

6. A lancet device according to claim 5, **characterized** in, that the loading mechanism comprises a block (38) rotably mounted on the tube end facing the socket (20) and having an outer contour mating with an inner contour in an independent part of the housing (21) comprising the socket (20) to be turned with this part of the housing, the tube (31) being provided at opposite sides with slots (41), into which protrude the ends of a pin (37) through the hammer (30), perpendicular to its axis and secured in the hammer (30) with equal parts protruding at each side of the hammer (30), and the block (38) being provided with corresponding slots (40), each of which at the position for the pin (37), when the hammer (30) is in its neutral position, pass over into a helical ramp (42) extending half the way around the block (38), the first part of the ramp (42) having guides for influencing the pin (37) in its longitudinal direction to centre the hammer (30) in the tube (31) before it is moved against the force of the shot spring (35) by the ends of the pin sliding upwards the ramps (42) when the block (38) is rotated on the tube (31).

7. A lancet device according to claim 5, **characterized** in that the adjustable means for limiting the movement of the hammer (30) comprise a sleeve (47) rotably mounted in the housing (21) coaxial with this housing at the end opposite the socket (20), the sleeve (47) being coupled to the button (22) to be rotated by turning this button and having a stop mechanism (50) to keep it in a selected rotary position, and that a bottom plate (46) is provided at the inner end of the sleeve (47), through which bottom plate the hammer (30) passes, this bottom plate (46) having on its side facing the inner of the sleeve (47) an angular stepped face with progressively increasing heighth of the steps, which face cooperates with a stop pin (50) on the hammer (30) inside the sleeve (47) to restrict the movement of the hammer (30) when released, each step corresponding to a selectable rotary position of the sleeve (47).

## Patentansprüche

1. Eine Lanzette-Vorrichtung, die ein Instrument mit einem Lanzette-Antriebshammer (30), der gespannt und ausgelöst werden kann, und eine Halteeinrichtung zur Halterung einer auswechselbaren Lanzette und einer Fingerplatte (1), mit einer Öffnung, durch die die Lanzette getrieben werden kann, wenn der Lanzette-Treiber ausgelöst wird, umfaßt, dadurch gekennzeichnet, daß die Lanzette und die Fingerplatte (1) in einer Lanzette-Einheit integriert sind, die in einer Fassung (20), die die Halteeinrichtung des Instruments bildet, aufzunehmen ist, wobei in dieser Einheit ein Stilett (4) mit einem spitzen vorderen Ende und einem hinteren Ende (5) federnd hinter der Fingerplatte (1) in einer Aufhängung aufgehängt ist, die starr verbunden ist mit dieser Fingerplatte (1) und die Verschiebung des Stiletts (4) in seiner axialen Richtung senkrecht zur Fingerplatte (1) erlaubt, so daß das spitze Ende angetrieben wird, aus der Vorderseite der Fingerplatte (1) auszutreten, wenn das Stilett (4) an seinem hinteren Ende (5) einen axialen Stoß von dem ausgelösten Lanzette-Antriebshammer (30) erhält, und in seine Anfangsposition hinter der Fingerplatte (1) zurückgezogen wird, wenn die Stoßeinwirkung aufgehört hat.

2. Eine Lanzette-Vorrichung nach Anspruch 1, dadurch gekennzeichnet, daß die federnde Aufhängung des Stiletts (4) mittels einer gewundenen Blattfeder (14, 15, 16) ausgebildet ist, deren Elastizität entlang des Blattes in der Art variiert, daß sichergestellt ist, daß das Stilett (4) nicht oder nur in geringem Maße von seiner Richtung senkrecht zu der Fingerplatte (1) abweicht, wenn es durch einen axialen Stoß auf sein hinteres Ende (5) beaufschlagt wird.

3. Eine Lanzette-Vorrichtung nach Anspruch 1 oder 2, dadurch gekennzeichnet, daß das Stilett (4) mit einer Schutzkappe (6) abgedeckt ist, die an der Stilettaufhängung befestigt ist und einen Griff (9) hat, der von der Vorderseite der Fingerplatte (1) zugänglich ist, um die Kappe (6) loszudrehen und sie vor Gebrauch zu entfernen, vorzugsweise nachdem die Lanzette-Einheit in einem Instrument angeordnet ist.

4. Eine Lanzette-Vorrichtung nach Anspruch 1, 2 oder 3, dadurch gekennzeichnet, daß die Fingerplatte (1) die Aufhängung und die Schutzkappe (6) als eine Einheit ausgeformt sind, in dem das Stilett (4) durch das Formverfahren eingebettet ist.

5. Eine Lanzette-Vorrichtung nach Anspruch 1, dadurch gekennzeichnet, daß das Instrument einen stabförmigen Lanzette-Antriebshammer (30), der in einem Rohr (31) koaxial zu einem Gehäuse (21) geführt wird, wobei das Rohr einen Durchmesser hat, der es erlaubt, daß der Hammerstab (30) schräg bezüglich der Achse des Rohrs (31) liegt; einen Lademechanismus, der den Hammer (30) weg vom Fassungsende des Gehäuses (21) gegen die Kraft einer Schußfeder (35) in eine im Rohr (31) zentrierte Position zwingt, wobei die Schußfeder (35) durch einen auslösbaren Klinkenmechanismus (53) gespannt und gehalten wird, wobei ein Ende des Hammers (30) in eine Bohrung (44) in einem Auslöseknopf (22) koaxial zum Gehäuse (21) am Ende gegenüber der Fassung (20) eingeschoben ist; einen Ausstoßer (43) innerhalb der Fassung (20), der einen Block umfaßt, der die Öffnung des Rohrs (31) an dem Ende, das der Fassung (20) zugewandt ist, und eine zentrale Öffnung (45) aufweist, durch die der Hammer (30) gerade hindurchpaßt, wenn er in dem Rohr (31) zentriert ist; eine Einrichtung, um den Hammer (30) in eine schräge Position in dem Rohr (31) zu zwingen, wobei ein Ende benachbart zum Ausstoßer-Block (43) neben seiner Öffnung (45) und das andere Ende benachbart zum inneren Ende des Auslöseknopfes (22) neben seiner Bohrung (44) liegt; und eine einstellbare Einrichtung, um die Bewegung des Hammers (30) in Längsrichtung zu begrenzen, wenn er ausgelöst wird, um einen Stoß auf das hintere Ende (5) des Stiletts (4) der in die Fassung (20) eingeschobenen Lanzette-Einheit auszuüben, umfaßt.

6. Eine Lanzette-Vorrichtung nach Anspruch 5, dadurch gekennzeichnet, daß der Lademechanismus einen Block (38) umfaßt, der drehbar auf dem der Fassung (20) zugewandten Ende des Rohrs angebracht ist und eine Außenkontur aufweist, die mit einer Innenkontur in einem unabhängigen, die Fassung (20) umfassenden Teil des Gehäuses (21) zusammenpaßt, der mit diesem Teil des Gehäuses zu drehen ist, wobei das Rohr (31) an gegenüberliegenden Seiten mit Schlitzen (41) ausgestattet ist, in die die Enden eines Stiftes (37) durch den Hammer (30), senkrecht zu seiner Achse und gesichert in dem Hammer (30) mit auf beiden Seiten des Hammers (30) gleich herausragenden Teilen, hineinragen, und der Block (38) mit entsprechenden Schlitzen (40) ausgestattet ist, von denen jeder an der Position für den Stift (37), wenn der Hammer (30) in seiner Ruhestellung ist, in eine spiralförmige Rampe (42) übergeht, die sich um den halben Block (38) erstreckt, wobei der erste Teil der Rampe (42) Führungen hat, um den Stift (37) in seiner Längsrichtung zu beeinflussen, um den Hammer (30) in dem Rohr (31) zu zentrieren, bevor er gegen die Kraft der Schußfeder (35) an den Enden des Stiftes bewegt wird, der die Rampe (42) hinaufgleitet, wenn der Block (38) auf dem Rohr (31) gedreht wird.

7. Eine Lanzette-Vorrichtung nach Anspruch 5, dadurch gekennzeichnet, daß die einstellbare Einrichtung zur Begrenzung der Bewegung des Hammers (30) einen Führungskörper (47) umfaßt, der drehbar im Gehäuse (21) koaxial zu diesem Gehäuse am Ende gegenüber der Fassung (20) angebracht ist, wobei der Führungskörper (47) mit dem Knopf (22) verbunden ist, so daß er durch Drehen dieses Knopfes gedreht werden kann, und einen Stop-Mechanismus (50) aufweist, um ihn in einer gewählten Drehposition zu halten, und daß eine Bodenplatte (46) am inneren Ende des Führungskörpers (47) vorgesehen ist, durch die der Hammer (30) hindurchtritt, wobei diese Bodenplatte (46) an ihrer Seite, die in das Innere des Führungskörpers (47) zeigt, eine ringförmige Stufenfläche mit progressiv zunehmender Höhe der Stufen aufweist, wobei diese Fläche mit einem Anschlagstift (50) an dem Hammer (30) innerhalb des Führungskörpers (47) zusammenwirkt, um die Bewegung des Hammers (30), wenn er ausgelöst wird, zu beschränken, wobei jede Stufe einer wählbaren Drehposition des Führungskörpers (47) entspricht.

## Revendications

1. Dispositif à lancette comprenant un outil comportant un marteau (30) d'entraînement de lancette qui peut être armé et libéré, et des moyens de retenue pour maintenir une lancette jetable et une plaque à doigt (1) comprenant une ouverture par laquelle la lancette peut être entraînée quand le dispositif d'entraînement de la lancette est libéré, caractérisé en ce que la lancette et la plaque à doigt (1) sont intégrées dans un unité à lancette prévue pour être reçue dans une douille (20) constituant le moyen de retenue de l'outil, unité dans laquelle un stylet (4) comprenant une extrémité avant pointue et une extrémité arrière (5) est suspendu de façon élastique à l'arrière de la plaque à doigt (1) dans une suspension reliée rigidement à la plaque à doigt (1) et permettant le déplacement du stylet (4) dans sa direction axiale perpendiculairement à la plaque à doigt (1) de manière que l'extrémité pointue soit entraînée et fasse saillie sur le côté avant de la plaque à doigt (1) quand le stylet (4) reçoit sur son extrémité arrière (5) un impact axial provenant du marteau (30) d'entraînement de lancette quand il est libéré, et est rétractée vers sa position initiale à l'arrière de la plaque à doigt (1) quand l'influence de l'impact a cessé.

2. Dispositif à lancette selon la revendication 1, caractérisé en ce que la suspension élastique du stylet (4) est formée par un ressort à lame en méandre (14, 15, 16), dont l'élasticité varie le long de la lame de manière que l'on soit assuré que le stylet (4) ne diverge pas, ou seulement d'une quantité mineure, de sa direction perpendiculaire à la plaque à doigt (1) quand il est soumis à l'influence d'un impact axial sur son extrémité arrière (5).

3. Dispositif à lancette selon la revendication 1 ou 2, caractérisé en ce que le stylet (4) est recouvert par un couvercle protecteur (6) qui est fixé à la suspension du stylet et comprend une nervure de saisie (9) accessible sur le côté avant de la plaque à doigt (1) pour libérer le couvercle (6) par torsion et le retirer avant l'utilisation, de préférence après que l'unité à lancette a été placée dans un outil.

4. Dispositif à lancette selon la revendication 1, 2 ou 3, caractérisé en ce que la plaque à doigt (1), la suspension et le couvercle protecteur (6) sont moulés d'un seul tenant, le stylet étant encastré pendant l'opération de moulage.

5. Dispositif à lancette selon la revendication 1, caractérisé en ce que l'outil comprend un marteau d'entraînement de lancette en forme de tige (30), guidé dans un tube (31) coaxialement à un logement (21), ce tube présentant un diamètre permettant à la tige (30) du marteau de se disposer en oblique par rapport à l'axe du tube (31); un mécanisme de chargement repoussant le marteau (30) de l'extrémité du logement (21) qui est tournée vers la douille à l'encontre de la force d'un ressort d'éjection (35) vers une position centrée dans le tube (31), le ressort d'éjection (35) étant tendu et maintenu par un mécanisme à cliquet libérable (53) tandis qu'une extrémité du marteau (30) est insérée dans un alésage (44) d'un bouton de commande (22) coaxial au logement (21) à l'extrémité opposée de la douille (20); un éjecteur (43) à l'intérieur de la douille (20) comprenant un bloc recouvrant l'orifice du tube (31) à l'extrémité qui est tournée vers la douille (20) et comprenant une ouverture centrale (45) par laquelle le marteau (30) peut juste passer quand il est centré dans le tube (31); des moyens pour forcer le marteau (30) à occuper une position en oblique dans le tube (31), avec une extrémité adjacente au bloc éjecteur (43) à côté de son ouverture (45), et l'autre extrémité adjacente de l'extrémité interne du bouton de commande (22) à côté de son trou (44); et des moyens réglables pour limiter le mouvement en longueur du marteau (30) quand il est libéré pour appliquer un impact sur l'extrémité arrière (5) du stylet (4) de l'unité de lancette insérée dans la douille (20).

6. Dispositif à lancette selon la revendication 5, caractérisé en ce que le mécanisme de chargement comprend un bloc (38) monté de façon rotative à l'extrémité du tube qui est face à la douille (20) et dont le contour externe est apparié au contour interne d'une partie indépendante du logement (21) qui comprend la douille (20) qui doit être tournée avec cette partie du logement, le tube (31) étant muni sur ses côtés opposés de fentes (41) par lesquelles font saillie les extrémités d'une tige (37) traversant le marteau (30), perpendiculairement à son axe et fixée au marteau (30) avec des parties de longueur égale faisant saillie de chaque côté du marteau (30), et le bloc (38) étant muni de fentes correspondantes (40), dont chacune, dans la position de la tige (37) quand le marteau (30) est dans sa position neutre, se prolonge par une rampe hélicoïdale (42) s'étendant sur la moitié du parcours autour du bloc (38), la première partie de la rampe (42) comprenant des guides pour influencer la tige (37) dans sa direction longitudinale en vue de centrer le marteau dans le tube (31) avant qu'il soit déplacé à l'encontre de la force du ressort d'éjection (35) par les extrémités de la tige qui coulisse vers le haut le long des rampes (42) quand le bloc (28) est tourné sur le tube (31).

7. Dispositif à lancette selon la revendication 5, caractérisé en ce que les moyens réglables pour limiter le mouvement du marteau (30) comprennent un manchon (47) monté de façon rotative dans le logement (21) coaxialement à ce logement à l'extrémité opposée à la douille (20), le manchon (47) étant accouplé au bouton (22) et étant entraîné en rotation quand on tourne ce bouton et comportant un mécanisme d'arrêt (50) pour le maintenir dans une position en rotation sélectionnée, et en ce qu'une plaque de fond (46) est prévue à l'extrémité interne du manchon (47), par laquelle passe la plaque de fond du marteau (30), cette plaque de fond (46) comprenant sur son côté qui est face à l'intérieur du manchon (47) une face à gradins angulaires dont les gradins ont une hauteur qui augmente progressivement, laquelle face coopère avec une tige d'arrêt (50) sur le marteau (30) à l'intérieur du manchon (47) pour limiter le mouvement du marteau (30) quand il est libéré, chaque gradin correspondant à une position en rotation sélectionnable du manchon (47).
